# EUROPEAN PATENT APPLICATION

(11) **EP 4 235 184 A1**
(43) Date of publication of application: **30.08.2023**
(21) Application number: 23158506.8
(22) Date of filing: 24.02.2023
(51) Int. Cl.: G01N 35/00

(54) **METHOD FOR APPLYING CONTROL REFERNCE VALUE TO SPECIMEN ANALYZER, SPECIMEN ANALYZER, AND COMPUTER PROGRAM**

(30) Priority: 28.02.2022 JP 2022030425
(71) Applicant: SYSMEX CORPORATION, Kobe-shi Hyogo 651-0073 (JP)
(72) Inventor: SHIRASUNA, Kei, Kobe-shi, Hyogo, 651-0073 (JP); FUKUMA, Daigo, Kobe-shi, Hyogo, 651-0073 (JP); NAGAO, Yuto, Kobe-shi, Hyogo, 651-0073 (JP); HAYASHI, Fumiaki, Kobe-shi, Hyogo, 651-0073 (JP); KISO, Kanako, Kobe-shi, Hyogo, 651-0073 (JP); KOHNO, Tomomi, Kobe-shi, Hyogo, 651-0073 (JP)
(74) Representative: Hoffmann Eitle

(57) **Abstract**

Disclosed is a method for applying a control reference value regarding a quality control sample to a specimen analyzer by a computer, the method comprising: generating the control reference value on the basis of a measurement result of the quality control sample measured by the specimen analyzer; and applying the control reference value to the specimen analyzer.

## Description

### CROSS REFERENCE TO RELATED APPLICATIONS

This application claims priority from prior Japanese Patent Application No. 2022-030425, filed on February 28, 2022, entitled "METHOD FOR APPLYING CONTROL REFERENCE VALUE TO SPECIMEN ANALYZER, SPECIMEN ANALYZER, AND COMPUTER PROGRAM", the entire content of which is incorporated herein by reference.

### FIELD OF THE INVENTION

The present invention relates to a method for applying a control reference value to a specimen analyzer, a specimen analyzer, and a computer program.

### BACKGROUND OF THE INVENTION

Conventionally, internal quality control using quality control samples has been conducted in test facilities. It is recommended that, in the internal quality control, desired values and margins for the quality control samples are individually determined in the test facilities as described in, for example, Kinns H, et al., "Internal quality control: best practice", September 18, 2016, J Clin Pathol 2013; 66: 1027-1032., doi: 10.1136/jclinpath-2013-201661. Therefore, when a person in charge in a test facility receives a quality control sample from a manufacturer, the person causes a specimen analyzer as a target of quality control to measure the quality control sample a plurality of times. The person inputs a plurality of measurement results having been obtained to a computer. The person causes the computer to calculate control reference values such as a desired value and a margin (allowable range) for the quality control sample by using commercially available spreadsheet software. The person inputs the obtained control reference values to the specimen analyzer as a target of quality control.

However, when the control reference values such as the desired value and the margin are individually determined and inputted to the specimen analyzer in the test facility, a problem arises in that it takes the person in charge an enormous amount of time to do so. In view of this, the control reference values are desired to be more efficiently determined and applied to the specimen analyzer in the test facility.

An object of the present invention is to cause a control reference value to be more efficiently determined and applied to a specimen analyzer in a test facility.

### SUMMARY OF THE INVENTION

A method of the present invention is a method for applying a control reference value regarding a quality control sample to a specimen analyzer by a computer. The method includes: generating the control reference value on the basis of a measurement result of the quality control sample measured by the specimen analyzer; and applying the control reference value to the specimen analyzer.

The method of the present invention eliminates the need for operations such as input of the measurement result of the quality control sample to the computer by a person in charge in a test facility and input of the obtained control reference value to the specimen analyzer by the person. Consequently, the control reference value can be more efficiently determined and applied to the specimen analyzer in the test facility.

A specimen analyzer of the present invention is a specimen analyzer to which a control reference value regarding a quality control sample is to be applied. The specimen analyzer includes: a measurement device; and a computer connected to the measurement device. The computer generates the control reference value on the basis of a measurement result of the quality control sample measured by the measurement device and applies the control reference value to the specimen analyzer.

The specimen analyzer of the present invention eliminates the need for operations such as input of the measurement result of the quality control sample to the computer by a person in charge in a test facility and input of the obtained control reference value to the specimen analyzer by the person. Consequently, the control reference value can be more efficiently determined and applied to the specimen analyzer in the test facility.

A computer program of the present invention is configured to cause a computer, configured to apply a control reference value regarding a quality control sample to a specimen analyzer, to execute: generating the control reference value on the basis of a measurement result of the quality control sample measured by the specimen analyzer; and applying the control reference value to the specimen analyzer.

The computer program of the present invention eliminates, through execution of the program, the need for operations such as input of the measurement result of the quality control sample to the computer by a person in charge in a test facility and input of the obtained control reference value to the specimen analyzer by the person. Consequently, the control reference value can be more efficiently determined and applied to the specimen analyzer in the test facility.

In the present invention, a control reference value can be more efficiently determined and applied to a specimen analyzer according to the facility.

### BRIEF DESCRIPTION OF THE DRAWINGS

FIG. 1 shows an overall configuration of a specimen analysis system;
FIG. 2 is a block diagram showing a configuration of a measurement device of each of specimen analyzers;
FIG. 3 is a block diagram showing a configuration of a computer;
FIG. 4A is a flowchart showing a process performed at the time of initial setting of rules of generating control reference values and/or at the time of changing the set rules;
FIG. 4B is a flowchart showing a process of, at the time of updating a quality control sample lot, registering a QC file, generating control reference values, and applying the control reference values to the specimen analyzers;
FIG. 4C is a flowchart showing a measurement process for a quality control sample;
FIG. 5 shows an example of the QC file stored in a storage unit;
FIG. 6 is a flowchart showing a subroutine of S 10 in FIG. 4A;
FIG. 7 shows an example of a screen displayed on a display at the time of selecting a basis analyzer and receiving a previous lot reference number;
FIG. 8 shows an example of a screen displayed on the display at the time of receiving selection of a measurement item that is a setting target for which a rule of generating a control reference value is to be set;
FIG. 9 shows an example of a screen displayed on the display at the time of receiving rules of generating a target value and a limit value;
FIG. 10 shows checkboxes (choices), texts each displayed in the column "TARGET" of a table when a corresponding one of the choices is selected, and rules of generating a target value;
FIG. 11A shows checkboxes (choices), texts each displayed in the column "LIMIT" of the table when a corresponding one of the checkboxes is selected, and rules of generating a limit value;
FIG. 11B shows checkboxes (choices), texts each displayed in the column "LIMIT" of the table when a corresponding one of the checkboxes is selected, and rules of generating a limit value;
FIG. 12 is a flowchart showing a subroutine of S24 in FIG. 4B;
FIG. 13 shows an example of a screen displayed on the display at the time of receiving selection of a measurement result;
FIG. 14 shows an example of a chart graph displaying mode in a reference value setting assistance screen displayed on the display;
FIG. 15 shows an example of a percentage graph displaying mode in the reference value setting assistance screen displayed on the display;
FIG. 16 shows an example of an internal quality control setting sheet displayed on the display; and
FIG. 17 shows an example of an internal quality control setting sheet displayed on the display.

### DETAILED DESCRIPTION

Hereinafter, a method for applying a control reference value to a specimen analyzer, a specimen analyzer, and a computer program according to an embodiment of the present disclosure will be described in detail with reference to the drawings.

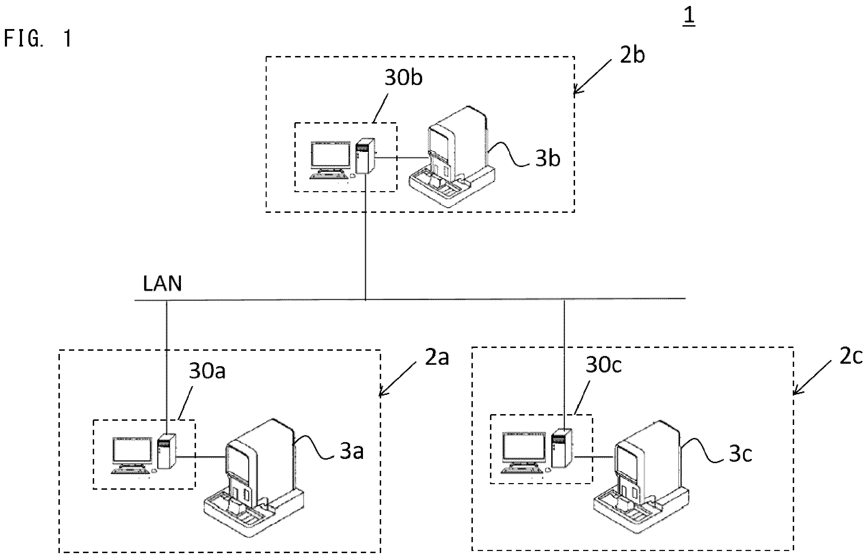

FIG. 1 shows an overall configuration of a specimen analysis system 1. The specimen analysis system 1 is disposed in a test facility such as a hospital or a test center. As shown in FIG. 1, the specimen analysis system 1 includes specimen analyzers 2a, 2b, and 2c. The specimen analyzer 2a includes a measurement device 3a and a computer 30a connected to the measurement device 3a. In the same manner as the specimen analyzer 2a, the specimen analyzer 2b includes a measurement device 3b and a computer 30b connected to the measurement device 3b. In the same manner as the specimen analyzer 2a, the specimen analyzer 2c includes a measurement device 3c and a computer 30c connected to the measurement device 3c. The computers 30a, 30b, and 30c are connected to each other via LAN cables so that communication therebetween can be performed. The specimen analyzers 2a, 2b, and 2c are each a blood cell counter for counting blood cells contained in a blood specimen. The specimen analyzers 2a, 2b, and 2c have configurations that are the same as one another, and thus the specimen analyzer 2a will be described below.

The specimen analyzer 2a is subjected to internal quality control by using a quality control sample. The quality control sample is a sample prepared so as to contain a predetermined component having a predetermined concentration and is produced by a manufacturer of the quality control sample. When a quality control sample of a new production lot is delivered from a manufacturer of the quality control sample to the test facility in which the specimen analysis system 1 is disposed, a person in charge (a user of the specimen analysis system 1) in the test facility causes the specimen analyzer 2a to measure the quality control sample one or more times and preferably a plurality of times. The computer 30a generates a control reference value regarding the quality control sample on the basis of a measurement result of the quality control sample and applies the control reference value to the specimen analyzer 2a. The control reference value generated on the basis of the measurement result of the quality control sample is a desired value (hereinafter, also referred to as a target value) and/or a value indicating an allowable range that are based on the measurement result of the quality control sample. The desired value is, for example: a measurement result obtained by measuring the quality control sample one time; or a statistical value such as the average value or the median value of a plurality of measurement results obtained by measuring the quality control sample a plurality of times. The value indicating the allowable range is, for example, the standard deviation of the plurality of measurement results or a value obtained by multiplying this standard deviation by a constant (hereinafter, referred to as a limit value (#)), and/or the coefficient of variation of the plurality of measurement results or a value obtained by multiplying this coefficient of variation by the constant (hereinafter, referred to as a limit value (%CV)). In the following descriptions, the limit value (#) and/or the limit value (%CV) is also referred to as a limit value. If the limit value (%CV) is calculated from measurement results of quality control samples of a plurality of production lots, the weighted average value of the coefficients of variation may be calculated on the basis of the number of days on which the quality control samples of the respective production lots are measured, and the calculated weighted average value may be used as the limit value (%CV). The user causes the specimen analyzer 2a to measure the quality control sample prior to measurement of a blood specimen obtained from a subject. A measurement result, of the quality control sample, obtained from the specimen analyzer 2a is outputted by the computer 30a. The user compares the measurement result and the control reference value applied to the specimen analyzer 2a with each other, and determines whether or not a reportable measurement result can be outputted from the specimen analyzer 2a.

A computer program (reference value applying program) for applying the control reference value to the specimen analyzer 2a is installed in the computer 30a. A method, for applying the control reference value to the specimen analyzer, that is performed by using the reference value applying program will be described later in detail.

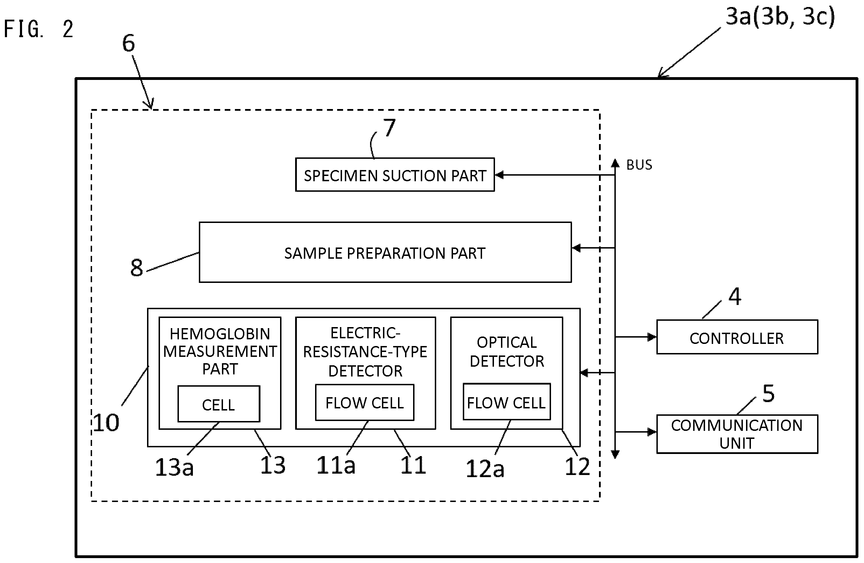

FIG. 2 is a block diagram showing a configuration of the measurement device 3a of the specimen analyzer 2a. The measurement device 3a includes a controller 4, a communication unit 5, and a measurement unit 6. The controller 4, the communication unit 5, and the measurement unit 6 are connected to each other via a bus. The communication unit 5 includes a communication interface for communication with the computer 30a. The communication interface is, for example, an Ethernet (registered trademark) interface. The controller 4 has an FPGA, a memory, and the like. The controller 4 controls operations of the communication unit 5 and each part of the measurement unit 6.

The measurement unit 6 prepares a measurement sample from a blood specimen and detects blood cells and hemoglobin contained in the measurement sample. The measurement unit 6 includes a specimen suction part 7, a sample preparation part 8, and a measurement part 10. The specimen suction part 7 suctions a blood specimen from a specimen container received by the specimen analyzer 2a and supplies the blood specimen to the sample preparation part 8. The sample preparation part 8 mixes the blood specimen and reagents with each other to prepare: a first measurement sample from which red blood cells and platelets are to be detected; a second measurement sample from which white blood cells are to be detected; and a third measurement sample from which hemoglobin is to be detected. The sample preparation part 8 supplies the samples to the measurement part 10.

The measurement part 10 includes an electric-resistance-type detector 11, an optical detector 12, and a hemoglobin measurement part 13. The electric-resistance-type detector 11 includes a flow cell 11a and detects, through a sheath flow DC detection method, red blood cells and platelets contained in the first measurement sample flowing in the flow cell 11a along with a sheath liquid. The optical detector 12 includes a flow cell 12a and detects, through flow cytometry, white blood cells contained in the second measurement sample flowing in the flow cell 12a along with a sheath liquid. The hemoglobin measurement part 13 includes a cell 13a and detects, through an SLS-hemoglobin method, hemoglobin contained in the third measurement sample accommodated in the cell 13a. Each of the detectors 11 and 12 and the measurement part 13 sends data indicating a measurement value of the corresponding sample to the controller 4. The controller 4 outputs the data to the computer 30a via the communication unit 5. Although the case where a blood specimen is measured has been described above, the measurement unit 6 performs the same operation also in a case where the quality control sample is measured. As the measurement unit 6, for example, a device described in U.S. Patent Publication No. 2016-0282377 can be used, and U.S. Patent Publication No. 2016-0282377 is hereby incorporated by reference.

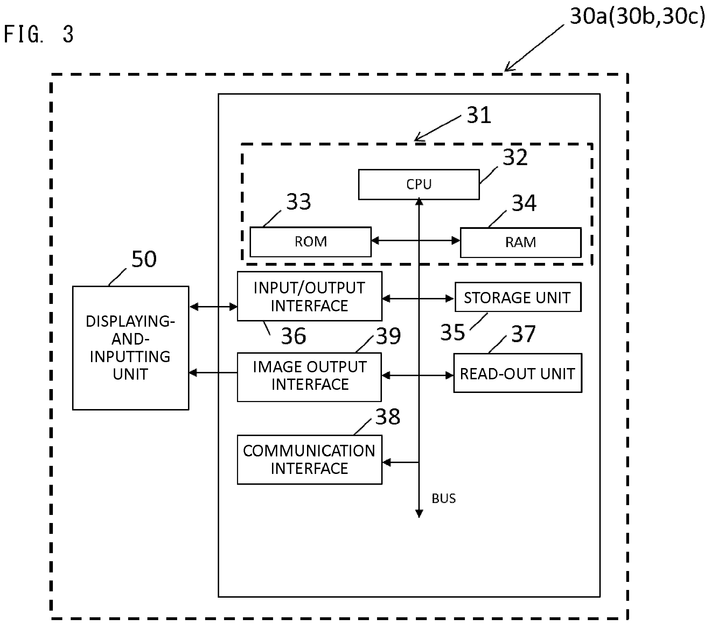

FIG. 3 is a block diagram showing a configuration of the computer 30a. The computer 30a includes a controller 31, a storage unit 35, an input/output interface 36, a read-out unit 37, a communication interface 38, an image output interface 39, and a displaying-and-inputting unit 50. The controller 31 includes a CPU 32, a ROM 33, and a RAM 34. The CPU 32, the ROM 33, the RAM 34, the storage unit 35, the input/output interface 36, the read-out unit 37, the communication interface 38, and the image output interface 39 are connected to each other via a bus so as to allow data communication therebetween. The displaying-and-inputting unit 50 includes a display, a keyboard, and a mouse.

The CPU 32 can execute a computer program stored in the ROM 33 and a computer program loaded into the RAM 34. The ROM 33 is implemented by a mask ROM, a PROM, an EPROM, an EEPROM, or the like and stores therein a computer program to be executed by the CPU 32 and data to be used for the computer program.

The RAM 34 is implemented by an SRAM, a DRAM, or the like. The RAM 34 is used as an operation region for the CPU 32 when computer programs stored in the ROM 33 and the storage unit 35 are executed.

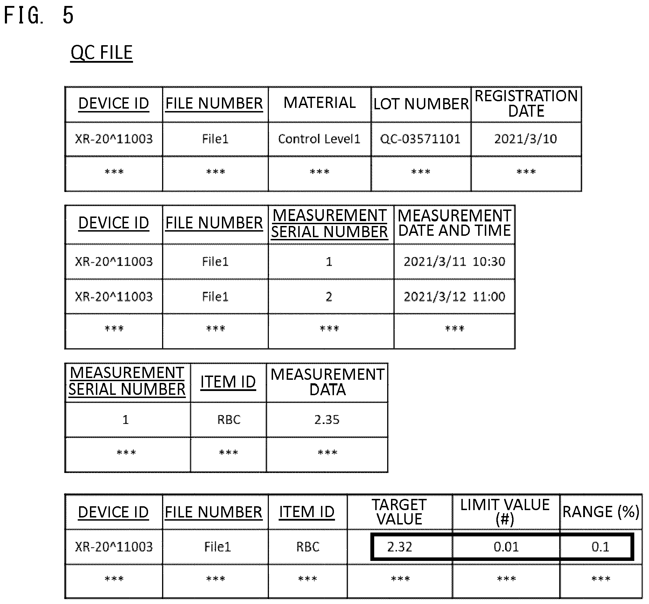

The storage unit 35 is implemented by a solid-state drive, a hard disk drive, or the like and stores therein: various computer programs to be executed by the CPU 32, such as an operating system and an application program; and data to be used for executing the computer programs. The storage unit 35 also stores therein the reference value applying program and data to be used for executing the reference value applying program. In addition, the storage unit 35 stores therein a QC file (FIG. 5) described later.

The read-out unit 37 is implemented by a flexible disk drive, a CD-ROM drive, a DVD-ROM drive, or the like and can read out a computer program or data stored in a portable storage medium.

The input/output interface 36 is implemented by, for example, an interface of USB, IEEE 1394, or the like. An inputting device composed of the keyboard and the mouse which are parts of the displaying-and-inputting unit 50 is connected to the input/output interface 36, and the user can input data to the controller 31 by using the inputting device.

The communication interface 38 is, for example, an Ethernet (registered trademark) interface. The controller 31 can transmit data to and receive data from the measurement device 3a and the computers 30b and 30c connected over a network by using a predetermined communication protocol via the communication interface 38.

The image output interface 39 is connected to the display of the displaying-and-inputting unit 50 and outputs, to the display, an image signal based on image data given from the CPU 32. The display displays an image on a screen according to the inputted image signal.

In the storage unit 35, for example, a Windows (registered trademark) operating system that provides a graphical user interface environment and that is manufactured by and available from Microsoft Corporation in the United States is installed. A case where the reference value applying program is executed on this operating system will be described below.

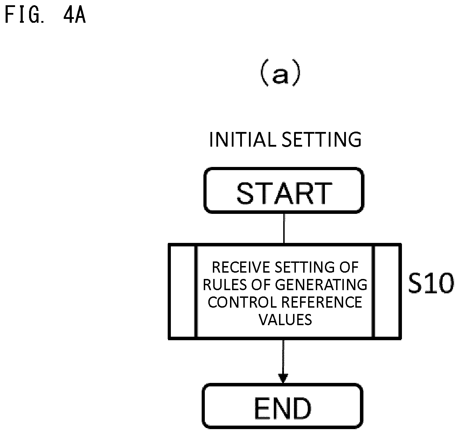

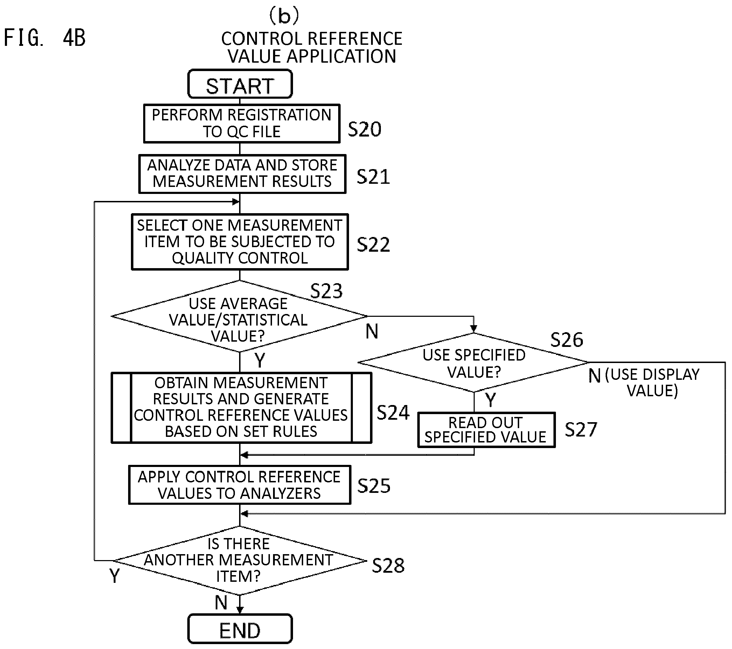

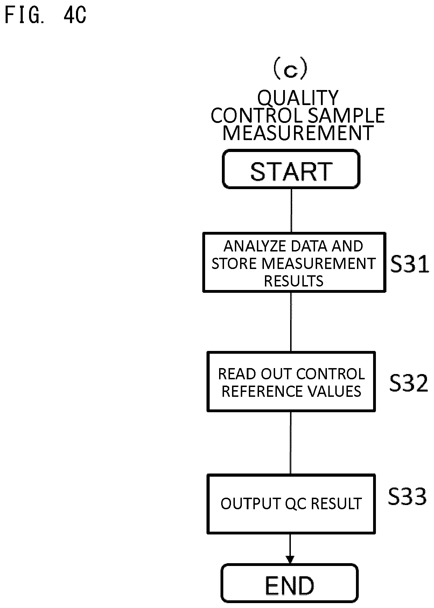

A method, for applying control reference values to the specimen analyzers 2a, 2b, and 2c, that is performed by the controller 31 of the computer 30a through execution of the reference value applying program will be specifically described below. FIG. 4A is a flowchart showing a process performed at the time of initial setting of rules of generating control reference values and/or at the time of changing the set rules. FIG. 4B is a flowchart showing a process of, at the time of updating a quality control sample lot, registering a QC file, generating control reference values, and applying the control reference values to the specimen analyzers 2a, 2b, and 2c. FIG. 4C is a flowchart showing a daily measurement process for a quality control sample.

As shown in FIG. 4A, the controller 31 of the computer 30a receives setting of rules of generating control reference values, at the time of initial setting of rules of generating control reference values and/or at the time of changing the set rules (S10). As the rules of generating control reference values, it is possible to set any of: (1) first rules of generating control reference values on the basis of measurement results obtained by measuring a quality control sample; (2) second rules of generating control reference values on the basis of values specified by the user; and (3) third rules of generating control reference values on the basis of values specified by the manufacturer of the quality control sample. A subroutine of the rule setting will be described later in detail.

The process shown in FIG. 4B is performed when a quality control sample of a new lot has been delivered to the test facility. The controller 31 of the computer 30a registers information about this new lot to a QC file stored in the storage unit 35 (S20). The controller 31 of the computer 30a reads out the information about the new lot from: a storage medium enclosed with the quality control sample; or a computer, of the manufacturer of the quality control sample or the specimen analyzer, that is connected to the computer 30a over the Internet. Then, the controller 31 registers the information to the QC file. Consequently, in the computer 30a, the QC file stored in the storage unit 35 is updated so as to include, as information thereof, the information about the new lot.

FIG. 5 shows an example of the QC file stored in the storage unit 35. In FIG. 5, the column "DEVICE ID" indicates identification information such as an identification number of each specimen analyzer. The column "MATERIAL" indicates the concentration level of the quality control sample, and the column "LOT NUMBER" indicates the lot number of the quality control sample. The column "MEASUREMENT SERIAL NUMBER" indicates a numerical value that is increased by one each time of measurement. The column "ITEM ID" indicates a measurement item. The columns "TARGET VALUE", "LIMIT VALUE (#)", and "RANGE (%)" respectively indicate a target value for the measurement value of the quality control sample, a limit value (#) for this measurement value, and limit value (#)÷target value.

When the processing in step S20 (FIG. 4B) is performed, numerical values (display values) specified by the manufacturer of the quality control sample are stored as the numerical values in the columns "TARGET VALUE", "LIMIT VALUE (#)", and "RANGE (%)" enclosed by thick rectangles in FIG. 5.

In execution of processing in step S21 by the controller 31 of the computer 30a, the user causes the specimen analyzer 2a to measure the quality control sample of the new lot one time or a plurality of times. Each time of measurement, the controller 31 of the computer 30a analyzes data indicating a measurement value, of the sample, received from the measurement device 3a and stores the result of the analysis into the storage unit 35 as a measurement result of the quality control sample (S21).

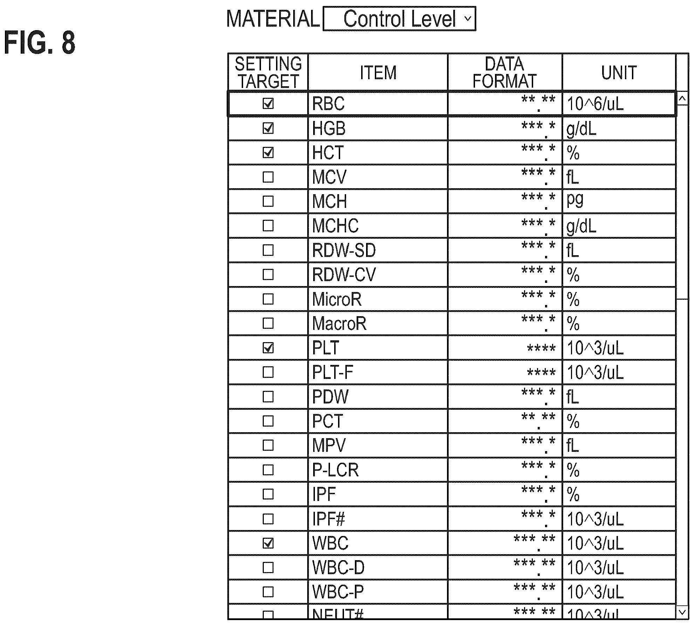

Then, the controller 31 of the computer 30a selects one measurement item from among measurement items to be subjected to quality control (for example, predetermined measurement items or measurement items selected, in the screen in FIG. 8 described later, as targets for which rules are to be set) (S22). The controller 31 of the computer 30a determines whether or not a rule of generating a control reference value by using an average value or a statistical value has been set for the selected measurement item (S23).

If the controller 31 of the computer 30a determines in step S23 that the rule of generating a control reference value by using an average value or a statistical value has been set for the selected measurement item (S23: Y), the controller 31 obtains the measurement results of the quality control sample and generates a control reference value, regarding the quality control sample of the new lot, for the specimen analyzers 2a, 2b, and 2c on the basis of the rule having been set in step S10 (S24). A subroutine of this step of generating a control reference value will be described later in detail.

Then, the controller 31 of the computer 30a applies the control reference value generated in step S24 to the specimen analyzers 2a, 2b, and 2c (S25). Specifically, the controller 31 of the computer 30a overwrites the target value, limit value (#), or limit value (#)÷target value for the specimen analyzer 2a having been generated in step S24 onto a corresponding one of the values in the columns "TARGET VALUE", "LIMIT VALUE", and "RANGE" (the values specified by the manufacturer of the quality control sample ) in the QC file stored in the storage unit 35. In addition, the controller 31 of the computer 30a writes the target value, limit value (#), or limit value (#)÷target value for the specimen analyzer 2a having been generated in step S24 into the corresponding one of the columns "TARGET VALUE", "LIMIT VALUE", and "RANGE" in the QC file stored in the storage unit 35 of each of the specimen analyzers 2b and 2c. Further, the controller 31 of the computer 30a may store, in the storage unit 35, the execution date and time of the applying of the control reference value and the name of the user who has performed the applying.

If the controller 31 of the computer 30a determines in step S23 that the rule of generating a control reference value by using an average value or a statistical value has not been set for the selected measurement item (S23: N), the controller 31 determines whether or not a rule of generating a control reference value by using a value specified by the user has been set for the selected measurement item (S26). If the controller 31 of the computer 30a determines in step S26 that the rule of generating a control reference value by using a value specified by the user has been set for the selected measurement item (S26: Y), the controller 31 reads out the value specified by the user from the storage unit 35 (S27). Then, the controller 31 of the computer 30a applies, as a control reference value, the value having been read out in step S27 to the specimen analyzers 2a, 2b, and 2c (S25). Specifically, the controller 31 of the computer 30a overwrites the target value (specified value), limit value (specified value (%))×target value, or limit value (specified value (%)) having been read out in step S27 onto the corresponding one of the values in the columns "TARGET VALUE", "LIMIT VALUE", and "RANGE" (the values specified by the manufacturer of the quality control sample) in the QC file stored in the storage unit 35 of each of the computers 30a, 30b, and 30c.

If the controller 31 of the computer 30a determines in step S26 that the rule of generating a control reference value by using a value specified by the user has not been set for the selected measurement item (S26: N "USE DISPLAY VALUE"), the controller 31 advances the process to step S28. In this case, a rule of using a value specified by the manufacturer of the quality control sample (display value) has been set for the selected measurement item. The value specified by the manufacturer of the quality control sample has already been stored in the corresponding one of the columns "TARGET VALUE", "LIMIT VALUE", and "RANGE" of the QC file through the QC file registration processing in step S20. Thus, the processing in step S25 is skipped.

After the processing in step S25 or if the result of the determination in step S26 is NO, the controller 31 of the computer 30a determines whether or not there is another measurement item to be subjected to quality control (S28). If the controller 31 of the computer 30a determines in step S28 that there is another measurement item to be subjected to quality control (S28: Y), the controller 31 returns the process to step S22. Meanwhile, if the controller 31 determines that there is no other measurement item to be subjected to quality control (S28: N), the controller 31 ends the process for applying a control reference value.

Ordinarily, after control reference values regarding the new lot is applied to the specimen analyzer 2a, the user causes the specimen analyzer 2a to measure the quality control sample of the new lot every day. As shown in FIG. 4C, the controller 31 of the computer 30a analyzes data indicating a measurement value, of the sample, received from the measurement device 3a and stores the result of the analysis into the storage unit 35 as a measurement result of the quality control sample (S31).

Then, the controller 31 of the computer 30a reads out the control reference values (the numerical values in the respective columns "TARGET VALUE", "LIMIT VALUE", and "RANGE") in the QC file stored in the storage unit 35 (S32).

Then, the controller 31 of the computer 30a outputs the control reference values having been read out and the measurement result of the quality control sample to the display of the displaying-and-inputting unit 50 as a QC result (S33).

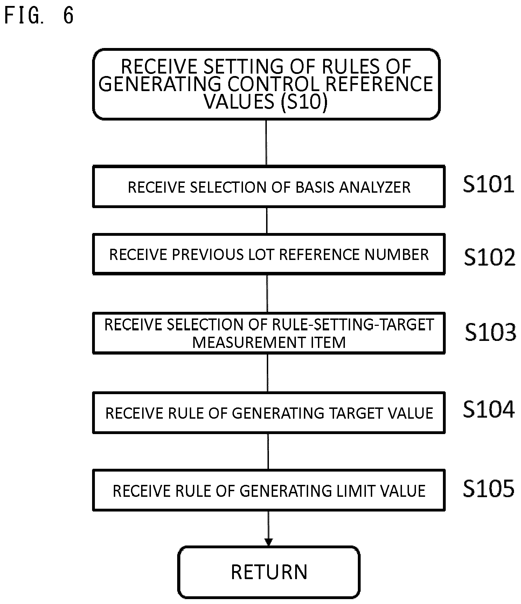

FIG. 6 shows a subroutine of the processing for receiving rule setting shown in step S10 in FIG. 4A. In this processing, the controller 31 of the computer 30a first receives selection of a basis analyzer on the basis of which quality control is to be performed among the specimen analyzers 2a, 2b, and 2c (S101). If no basis analyzer is selected, the processing in step S101 can be omitted. Then, if measurement results of quality control samples of previous production lots (previous lots) are used to calculate a control reference value (for example, a limit value (#) and/or a limit value (%CV)), the controller 31 of the computer 30a receives the number (reference number) of the previous lots to be used for the calculation (S102). The processing in step S101 and the processing in step S 102 may be performed according to an inverted sequence or may be performed simultaneously.

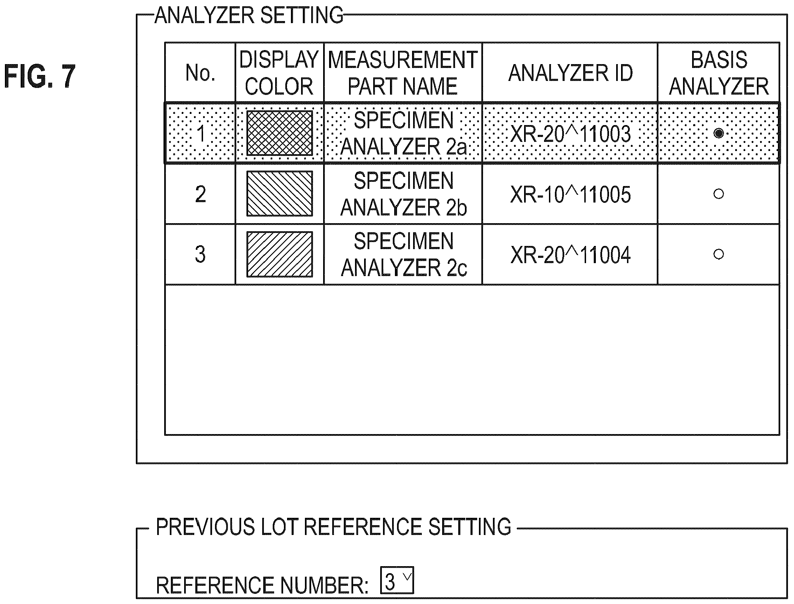

FIG. 7 shows an example of a screen displayed on the display of the displaying-and-inputting unit 50 at the time of selecting a basis analyzer in step S101 and receiving a previous lot reference number in step S102. In this screen, the column "MEASUREMENT PART NAME" indicates names given to the specimen analyzers 2a, 2b, and 2c by the user in advance, and the column "ANALYZER ID" indicates identification information about the specimen analyzers 2a, 2b, and 2c. A basis analyzer is selected by selecting one of checkboxes in the column "BASIS ANALYZER" through operation of the mouse or the keyboard of the displaying-and-inputting unit 50.

The previous lot reference number can be selected by selecting a numerical value from a pull-down menu in the field "PREVIOUS LOT REFERENCE SETTING" through operation of the mouse or the keyboard of the displaying-and-inputting unit 50. FIG. 7 shows an example in which: the "specimen analyzer 2a" has been selected as a basis analyzer; and 3 which is the number of previous lots is selected as a previous lot reference number.

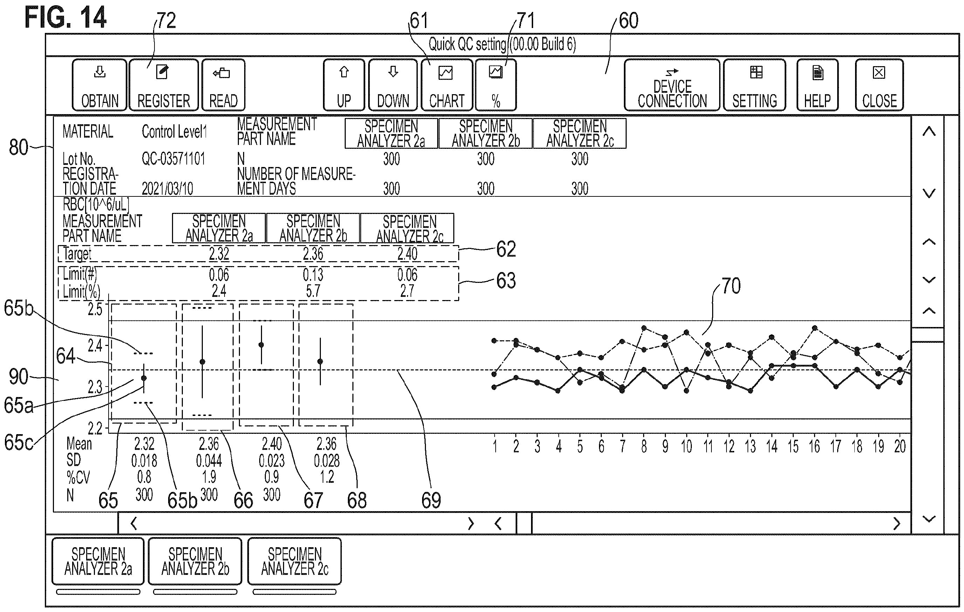

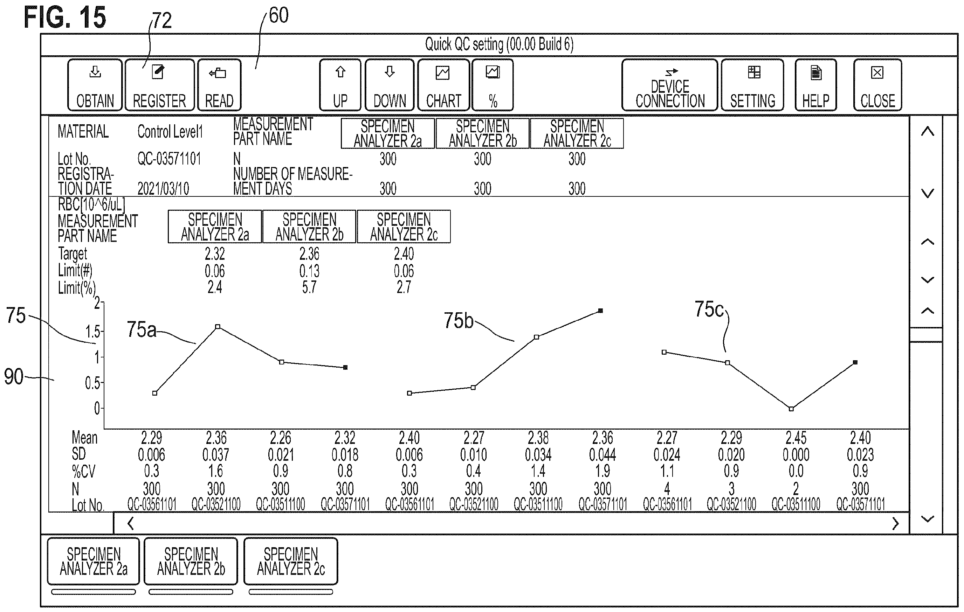

In the column "DISPLAY COLOR" in FIG. 7, different colors are specified for the respective specimen analyzers 2a, 2b, and 2c, and colors of dots and lines displayed correspondingly to the respective specimen analyzers are shown in order to easily distinguish the specimen analyzers 2a, 2b, and 2c from one another in a reference value setting assistance screen (FIGS. 14 and 15) described later.

With reference back to FIG. 6, after step S 102, the controller 31 of the computer 30a receives selection of a measurement item for which a rule of generating a control reference value is to be set (S103). The measurement item having been selected here is a setting target for which a rule of generating a control reference value is to be set. In step S 103, a measurement item that is a setting target for which a rule of generating a control reference value is to be set is selected for each of concentration levels of the quality control sample.

FIG. 8 shows an example of a screen displayed on the display of the displaying-and-inputting unit 50 at the time of receiving selection of a measurement item that is a setting target for which a rule of generating a control reference value is to be set. The field "MATERIAL" is a field for selecting a concentration level of the quality control sample. For the quality control sample, for example, a low concentration level, an intermediate concentration level, or a high concentration level can be set. "Control Level 1" indicates the low concentration level, "Control Level 2" indicates the intermediate concentration level, and "Control Level 3" indicates the high concentration level. In FIG. 8, "Control Level 1" indicating the low concentration level is selected. In this state, selection of a measurement item that corresponds to "Control Level 1" and that is a setting target for which a rule of generating a control reference value is to be set, is received. The column "ITEM" indicates measurement items for the specimen analyzer 2a. When a checkbox in the column "SETTING TARGET" is selected through operation of the mouse or the keyboard of the displaying-and-inputting unit 50, the corresponding measurement item is selected as a setting target for which a rule of generating a control reference value is to be set. In FIG. 8, an RBC (red blood cell count), an HGB (the amount of hemoglobin), an HCT (hematocrit value), a PLT (platelet count), and a WBC (white blood cell count) are selected as setting targets for which rules of generating control reference values are to be set.

With reference back to FIG. 6 again, after step S103, the controller 31 of the computer 30a receives setting of a rule of generating a target value (S104) and then receives setting of a rule of generating a limit value (S105). In steps S104 and S105, setting of rules of generating a target value and a limit value is received for each of the concentration levels of the quality control sample. The processing in step S104 and the processing in S105 may be performed according to an inverted sequence or may be performed simultaneously.

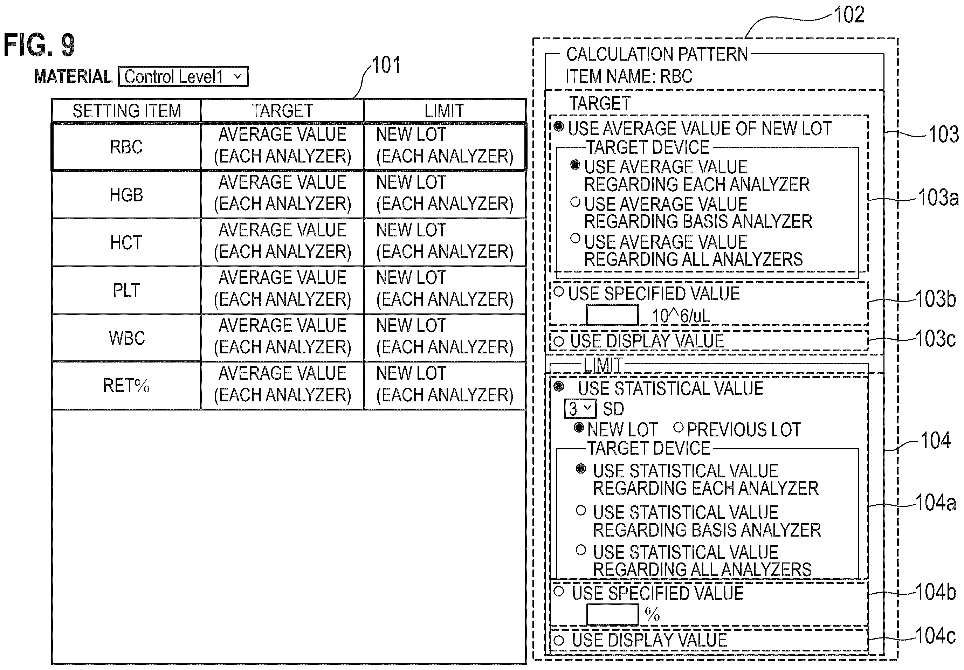

FIG. 9 shows an example of a screen displayed on the display of the displaying-and-inputting unit 50 at the time of receiving setting of rules of generating a target value and a limit value. In this screen as well, a concentration level of the quality control sample is selected in the field "MATERIAL" in the same manner as in the screen shown in FIG. 8.

On the screen in FIG. 9, a table 101 including the columns "SETTING ITEM", "TARGET", and "LIMIT", and a region 102 of "CALCULATION PATTERN", are displayed. In the column "SETTING ITEM" of the table 101, all of the measurement items selected in step S103 are displayed. For each of the measurement items, a corresponding rule of generating a target value and a corresponding rule of generating a limit value are respectively displayed in the column "TARGET" and the column "LIMIT" of the table 101. The table 101 is configured to allow selection of any one of rows through operation of the mouse or the keyboard of the displaying-and-inputting unit 50, and the selected row is displayed in a color different from the colors of the other rows. In the example in FIG. 9, the row in which the measurement item is RBC is selected. In the region 102 of "CALCULATION PATTERN", the measurement item (in the example in FIG. 9, RBC) in the row selected in the table 101 is displayed as an item name. That is, setting of generation rules can be received for each of the measurement items in the screen in FIG. 9.

The region 102 of "CALCULATION PATTERN" includes: a region 103 in which setting of a rule of generating a target value is received; and a region 104 in which setting of a rule of generating a limit value is received. The region 103 related to target value includes: a region 103a in which setting of a rule of generating a target value by using an average value of the new lot is received; a region 103b in which setting of a rule of generating a target value by using a value specified by the user is received; and a region 103c in which setting of a rule of generating a target value by using a value specified by the manufacturer of the quality control sample is received. The region 104 related to limit value includes: a region 104a in which setting of a rule of generating a limit value by using a statistical value of the new lot or a previous lot is received; a region 104b in which setting of a rule of generating a limit value by using a value specified by the user is received; and a region 104c in which setting of a rule of generating a limit value by using a value specified by the manufacturer of the quality control sample is received.

As the rule of generating a target value, it is possible to select one of: a (1) first rule of generating a control reference value on the basis of measurement results obtained by measuring the quality control sample; a (2) second rule of generating a control reference value on the basis of a value specified by the user; and a (3) third rule of generating a control reference value on the basis of a value specified by the manufacturer of the quality control sample. If the first rule is to be set, the user selects the checkbox of (1) "USE AVERAGE VALUE OF NEW LOT". If the second rule is to be set, the user selects the checkbox of (2) "USE SPECIFIED VALUE". If the third rule is to be set, the user selects the checkbox of (3) "USE DISPLAY VALUE". If the user selects (1) "USE AVERAGE VALUE OF NEW LOT", the user can further select, as a target device(s), one of: the checkbox of (1a) "USE AVERAGE VALUE REGARDING EACH ANALYZER"; the checkbox of (1b) "USE AVERAGE VALUE REGARDING BASIS ANALYZER"; and the checkbox of (1c) "USE AVERAGE VALUE REGARDING ALL ANALYZERS". In this manner, the rule of generating a target value can be set by selecting any of the checkboxes (choices). If the checkbox of (1) "USE AVERAGE VALUE OF NEW LOT" is selected, the result of the determination in the processing in step S23 (FIG. 4B) is YES. If the checkbox of (2) "USE SPECIFIED VALUE" is selected, the result of the determination in step S26 is YES. If the checkbox of (3) "USE DISPLAY VALUE" is selected, the result of the determination in step S26 is NO.

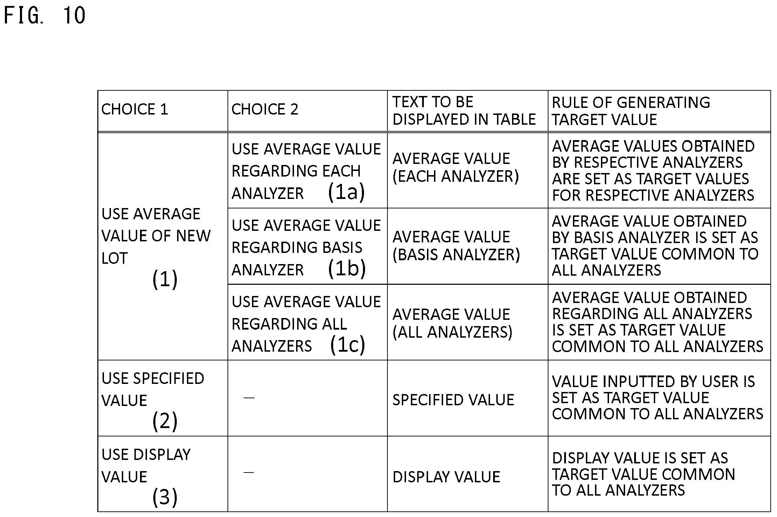

FIG. 10 shows the checkboxes (choices), texts each displayed in the column "TARGET" of the table 101 when a corresponding one of the checkboxes is selected, and the rules of generating a target value. Specifically, if the above checkboxes of (1) and (1a) are selected, the text "AVERAGE VALUE (EACH ANALYZER)" is displayed in the column "TARGET" of the table 101 in FIG. 9. In this case, each of the specimen analyzers 2a, 2b, and 2c measures the quality control sample of the new lot a plurality of times, and each of the computers 30a, 30b, and 30c performs the processing in step S24 (FIG. 4B) on the basis of a plurality of measurement results obtained by the corresponding one of the specimen analyzers 2a, 2b, and 2c. Consequently, individual target values are generated for the respective specimen analyzers 2a, 2b, and 2c.

If the above checkboxes of (1) and (1b) are selected, the text "AVERAGE VALUE (BASIS ANALYZER)" is displayed in the column "TARGET" of the table 101 in FIG. 9. In this case, a specimen analyzer selected as a basis analyzer in the screen in FIG. 7 (in the example in FIG. 7, the specimen analyzer 2a) measures the quality control sample of the new lot a plurality of times, and the corresponding computer 30a performs the processing in step S24 (FIG. 4B) on the basis of a plurality of measurement results obtained by the specimen analyzer 2a. Consequently, a target value to be applied in common to the specimen analyzers 2a, 2b, and 2c is generated.

If the above checkboxes of (1) and (1c) are selected, the text "AVERAGE VALUE (ALL ANALYZERS)" is displayed in the column "TARGET" of the table 101 in FIG. 9. In this case, each of the specimen analyzers 2a, 2b, and 2c measures the quality control sample of the new lot a plurality of times, and the computer 30a performs the processing in step S24 (FIG. 4B) on the basis of all of measurement results obtained by the specimen analyzers 2a, 2b, and 2c. Consequently, a target value to be applied in common to the specimen analyzers 2a, 2b, and 2c is generated.

If the above checkbox of (2) is selected, a value inputted by the user in the region 103b through operation of the mouse or the keyboard of the displaying-and-inputting unit 50 is displayed in the column "TARGET" of the table 101 in FIG. 9. In this case, the value displayed in the column "TARGET" is a target value to be applied in common to the specimen analyzers 2a, 2b, and 2c.

If the above checkbox of (3) is selected, a target value specified by the manufacturer of the quality control sample is displayed in the column "TARGET" of the table 101 in FIG. 9. The target value specified by the manufacturer of the quality control sample is listed in the column "TARGET VALUE" of the QC file (FIG. 5) stored in the storage unit 35.
In this case, the value displayed in the column "TARGET" is a target value to be applied in common to the specimen analyzers 2a, 2b, and 2c.

As the rule of generating a limit value, it is possible to select one of: a (4) first rule of generating a control reference value on the basis of measurement results obtained by measuring the quality control sample; a (5) second rule of generating a control reference value on the basis of a value specified by the user; and a (6) third rule of generating a control reference value on the basis of a value specified by the manufacturer of the quality control sample. If the first rule is to be set, the user selects the checkbox of (4) "USE STATISTICAL VALUE". If the second rule is to be set, the user selects the checkbox of (5) "USE SPECIFIED VALUE". If the third rule is to be set, the user selects the checkbox of (6) "USE DISPLAY VALUE". If the user selects (4) "USE STATISTICAL VALUE", it is possible to further select one of the checkbox of (4a) "NEW LOT" and the checkbox of (4b) "PREVIOUS LOT" in order to select whether to use, as measurement results to be used for calculation of a limit value, measurement results of the quality control sample of the new lot to perform the calculation or measurement results of a quality control sample of a previous lot to perform the calculation. It is possible to further select, as a target device(s), one of: the checkbox of (4a1) or (4b 1) "USE STATISTICAL VALUE REGARDING EACH ANALYZER"; the checkbox of (4a2) or (4b2) "USE STATISTICAL VALUE REGARDING BASIS ANALYZER"; and the checkbox of (4a3) or (4b3) "USE STATISTICAL VALUE REGARDING ALL ANALYZERS". In this manner, the rule of generating a limit value can be set by selecting any of the checkboxes (choices). If the checkbox of (4) "USE STATISTICAL VALUE" is selected, the result of the determination in the processing in step S23 (FIG. 4B) is YES. If the checkbox of (5) "USE SPECIFIED VALUE" is selected, the result of the determination in step S26 is YES. If the checkbox of (6) "USE DISPLAY VALUE" is selected, the result of the determination in step S26 is NO.

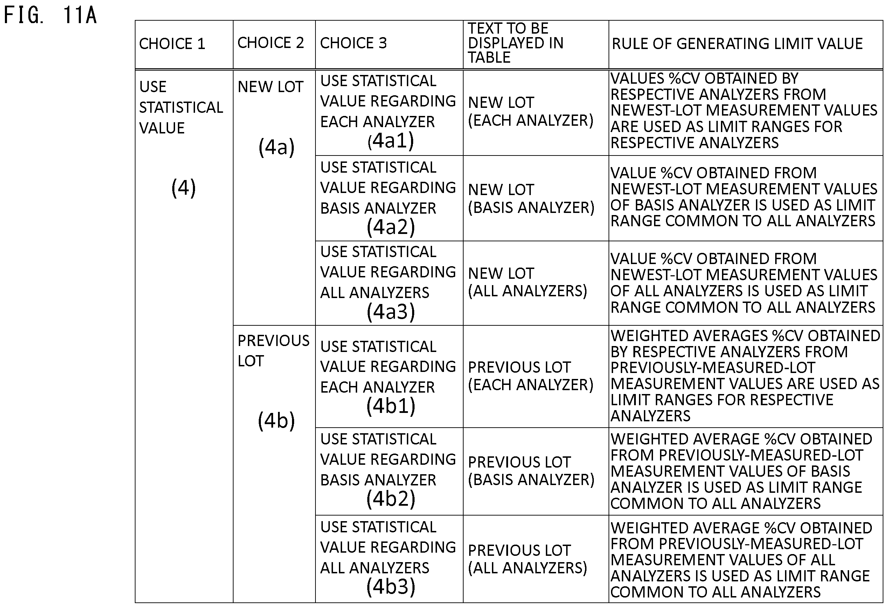

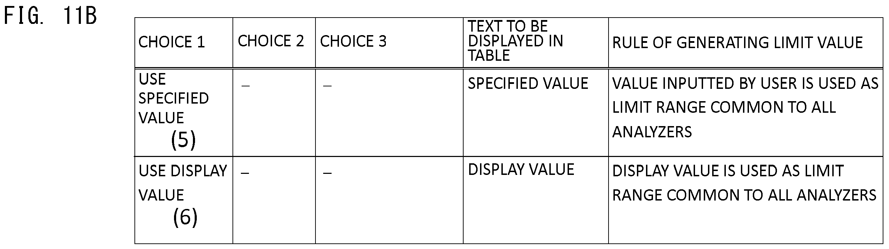

FIGS. 11A and 11B show the checkboxes (choices), texts each displayed in the column "LIMIT" of the table 101 when a corresponding one of the checkboxes is selected, and the rules of generating a limit value. Specifically, if the above checkboxes of (4), (4a), and (4a1) are selected, the text "NEW LOT (EACH ANALYZER)" is displayed in the column "LIMIT" of the table 101 in FIG. 9. In this case, each of the specimen analyzers 2a, 2b, and 2c measures the quality control sample of the new lot a plurality of times, and each of the computers 30a, 30b, and 30c performs the processing in step S24 (FIG. 4B) on the basis of a plurality of measurement results obtained by the corresponding one of the specimen analyzers 2a, 2b, and 2c. Consequently, individual limit values are generated for the respective specimen analyzers 2a, 2b, and 2c.

If the above checkboxes of (4), (4a), and (4a2) are selected, the text "NEW LOT (BASIS ANALYZER)" is displayed in the column "LIMIT" of the table 101 in FIG. 9. In this case, a specimen analyzer selected as a basis analyzer in the screen in FIG. 7 (in the example in FIG. 7, the specimen analyzer 2a) measures the quality control sample of the new lot a plurality of times, and the corresponding computer 30a performs the processing in step S24 (FIG. 4B) on the basis of a plurality of measurement results obtained by the specimen analyzer 2a. Consequently, a limit value to be applied in common to the specimen analyzers 2a, 2b, and 2c is generated.

If the above checkboxes of (4), (4a), and (4a3) are selected, the text "NEW LOT (ALL ANALYZERS)" is displayed in the column "LIMIT" of the table 101 in FIG. 9. In this case, each of the specimen analyzers 2a, 2b, and 2c measures the quality control sample of the new lot a plurality of times, and the computer 30a performs the processing in step S24 (FIG. 4B) on the basis of all of measurement results obtained by the specimen analyzers 2a, 2b, and 2c. Consequently, a limit value to be applied in common to the specimen analyzers 2a, 2b, and 2c is generated.

If the above checkboxes of (4), (4b), and (4b 1) are selected, the text "PREVIOUS LOT (EACH ANALYZER)" is displayed in the column "LIMIT" of the table 101 in FIG. 9. In this case, the same process as that in the case where the checkboxes of (4), (4a), and (4a1) are selected is performed on the basis of measurement results of a quality control sample of a previous lot. Consequently, individual limit values are generated for the respective specimen analyzers 2a, 2b, and 2c.

If the above checkboxes of (4), (4b), and (4b2) are selected, the text "PREVIOUS LOT (BASIS ANALYZER)" is displayed in the column "LIMIT" of the table 101 in FIG. 9. In this case, the same process as that in the case where the checkboxes of (4), (4a), and (4a2) are selected is performed on the basis of the measurement results of the quality control sample of the previous lot. Consequently, a limit value to be applied in common to the specimen analyzers 2a, 2b, and 2c is generated.

If the above checkboxes of (4), (4b), and (4b3) are selected, the text "PREVIOUS LOT (ALL ANALYZERS)" is displayed in the column "LIMIT" of the table 101 in FIG. 9. In this case, the same process as that in the case where the checkboxes of (4), (4a), and (4a3) are selected is performed on the basis of the measurement results of the quality control sample of the previous lot. Consequently, a limit value to be applied in common to the specimen analyzers 2a, 2b, and 2c is generated.

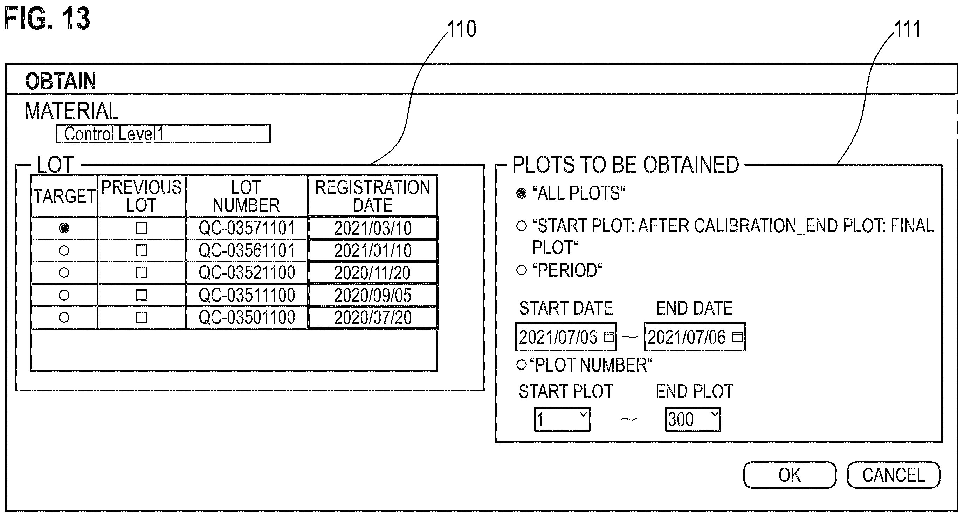

The number of previous lots of quality control samples to be used in generation of a limit value is set according to the numerical value selected in the field "REFERENCE NUMBER" in FIG. 7 (if the checkbox of "PREVIOUS LOT" is selected in a screen in FIG. 13 described later, the said number is set according to this selection).

If the user selects (4) "USE STATISTICAL VALUE", mSD (m represents a numerical value that can be selected from among 1, 2, 3, and 4) is also displayed under the field "USE STATISTICAL VALUE" of the region 104a in FIG. 9. If the user selects a numerical value of "m" through operation of the mouse or the keyboard of the displaying-and-inputting unit 50, selection can be made such that a value obtained by multiplying a standard deviation by "m" and a value obtained by multiplying a coefficient of variation by "m" are determined as the limit value (#) and the limit value (%CV). For example, 3SD corresponds to a standard deviation of 3σ.

If the above checkbox of (5) is selected, a value inputted by the user in the region 104b through operation of the mouse or the keyboard of the displaying-and-inputting unit 50 is displayed in the column "LIMIT" of the table 101 in FIG. 9. In this case, the value displayed in the column "LIMIT" is a limit value to be applied in common to the specimen analyzers 2a, 2b, and 2c.

If the above checkbox of (6) is selected, a limit value (#) specified by the manufacturer of the quality control sample is displayed in the column "LIMIT" of the table 101 in FIG. 9. The limit value (#) specified by the manufacturer of the quality control sample is described in the column "LIMIT VALUE" of the QC file (FIG. 5) stored in the storage unit 35. In this case, the value displayed in the column "LIMIT" is a limit value to be applied in common to the specimen analyzers 2a, 2b, and 2c.

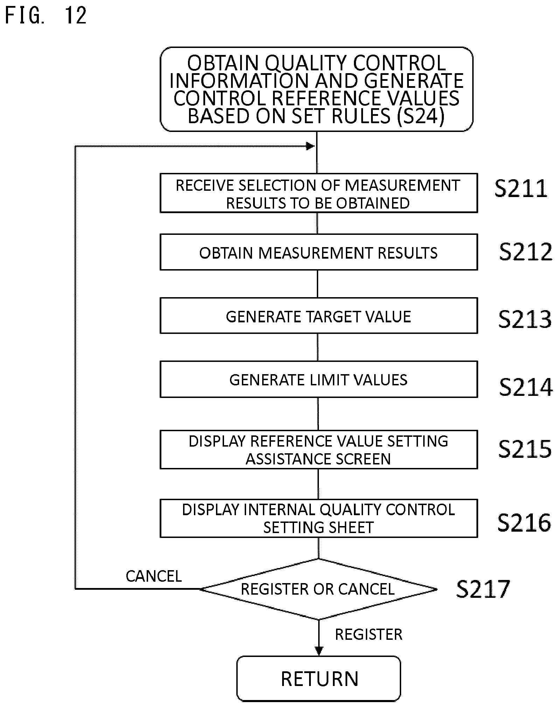

FIG. 12 shows a subroutine of S24 in FIG. 4B. As described with reference to the above FIG. 4B, if the quality control sample of the new lot is registered, the controller 31 of the computer 30a obtains measurement results of the quality control sample and generates control reference values on the basis of the rules having been set in S10 in FIG. 4A, in S24.

The controller 31 of the computer 30a receives selection of measurement results, of the quality control sample, to be used for generating control reference values (S211). FIG. 13 shows an example of a screen displayed on the display of the displaying-and-inputting unit 50 at the time of receiving selection of measurement results. The field "MATERIAL" is a field in which a concentration level of the quality control sample is selected. Consequently, measurement results can be selected for each of the concentration levels of the quality control sample. In a table 110 of "LOT", quality control samples for which measurement results have been stored are displayed for respective lot numbers in a descending order from the latest registration date. The quality control sample displayed in the first row and having a lot number of QC-03571 101 is of the new lot, and the other quality control samples are of previous lots. In this display, measurement results of a quality control sample for which the corresponding checkbox in the column "TARGET" or "PREVIOUS LOT" has been selected are selected. If "NEW LOT" has been selected in the region 104a of the screen in FIG. 9, the screen in FIG. 13 is displayed in a state where only a checkbox, in the column "TARGET", corresponding to the quality control sample that is of the new lot and that has a lot number of QC-03571101 is selected. If "PREVIOUS LOT" has been selected in the region 104a of the screen in FIG. 9, the screen in FIG. 13 is displayed in a state where checkboxes in the column "PREVIOUS LOT" are selected according to the reference number selected in the field "REFERENCE NUMBER" in FIG. 7. The screen in FIG. 13 indicates an example in which "NEW LOT" has been selected in the region 104a of the screen in FIG. 9. If the user desires to change the selection, the selection can be changed by selecting a checkbox in the column "TARGET" or "PREVIOUS LOT" through operation of the mouse or the keyboard of the displaying-and-inputting unit 50.

A region 111 of "PLOTS TO BE OBTAINED" in FIG. 13 is a region in which measurement results to be used for generating control reference values are selected from among the measurement results of the quality control sample selected in the table 110 of "LOT". If the checkbox of "ALL PLOTS" is selected, all of the measurement results of the quality control sample selected in the table 110 of "LOT" are used for generating control reference values. If the checkbox of "START PLOT: AFTER CALIBRATION END PLOT: FINAL PLOT" is selected, measurement results that are obtained after calibration processing is performed on the specimen analyzers 2a, 2b, and 2c having performed measurement among the measurement results of the quality control sample selected in the table 110 of "LOT" are used for generating control reference values. If the checkbox of "PERIOD" is selected, measurement results that are obtained in a period from a date inputted in the field "START DATE" to a date inputted in the field "END DATE" among the measurement results of the quality control sample selected in the table 110 of "LOT" are used for generating control reference values. If the checkbox of "PLOT NUMBER" is selected, measurement results that range from a numerical value inputted in the field "START PLOT" to a numerical value inputted in the field "END PLOT" among the measurement results of the quality control sample selected in the table 110 of "LOT" are used for generating control reference values. For example, in the example in FIG. 13, measurement results that range from the measurement result obtained for the first time to the measurement result obtained for the three hundredth time are used for generating control reference values.

If an OK button is selected after selection of checkboxes in FIG. 13 is completed, the controller 31 of the computer 30a reads out the measurement results from the storage unit 35 according to the selected checkboxes in step S212 in FIG. 12. If the selected measurement results are not stored in the storage unit 35 of the computer 30a, the controller 31 of the computer 30a requires the computers 30b and 30c of the other specimen analyzers 2b and 2c to transmit the measurement results of the quality control sample necessary for generating control reference values and receives the measurement results therefrom. For example, if the above checkboxes of (1) and (1a), (1) and (1c), (4), (4a) and (4a1), or (4), (4a), and (4a3) are selected, the controller 31 of the computer 30a requires the computers 30b and 30c to transmit the measurement results of the quality control sample necessary for generating control reference values and receives the measurement results therefrom.

Then, the controller 31 of the computer 30a generates a target value (S213). The controller 31 of the computer 30a calculates, on the basis of the corresponding rule the setting of which has been received in step S10, the average value of the plurality of measurement results obtained in step S212, thereby generating a target value (S213). Then, the controller 31 of the computer 30a generates limit values (S214). The controller 31 of the computer 30a calculates, on the basis of the corresponding rule the setting of which has been received in step S10, the standard deviation and the coefficient of variation of the plurality of measurement results obtained in step S212, thereby generating limit values (S214). The sequence of performing the processing in S213 and the processing in S214 may be inverted.

Then, in S215 in FIG. 12, the controller 31 of the computer 30a causes the display of the displaying-and-inputting unit 50 to display a reference value setting assistance screen including the target value and the limit values (control reference values) generated in steps S213 and S214. An initial screen of the reference value setting assistance screen is displayed in a chart graph displaying mode shown in FIG. 14, and the mode can be switched to a percentage graph displaying mode shown in FIG. 15. FIG. 14 shows an example of the reference value setting assistance screen displayed in the chart graph displaying mode. The reference value setting assistance screen can be displayed for each of the concentration levels of the quality control sample and for each of the measurement items. The screen in FIG. 14 shows an example in which: the quality control sample is of the new lot; the concentration level of the quality control sample is "Control Level 1"; the measurement item is RBC; and the rule of using an average value regarding each analyzer and the rule of using a statistical value regarding each analyzer have been selected as rules of generating control reference values. The reference value setting assistance screen includes: a tool bar region 60 at an upper part of the screen; a material region 80 located downward of the tool bar region 60; and a chart graph region 90 located downward of the material region 80. The tool bar region 60 and the material region 80 are screen regions common to the chart graph displaying mode and the percentage graph displaying mode. That is, content displayed in each of the tool bar region 60 and the material region 80 is not changed even upon switching between the modes.

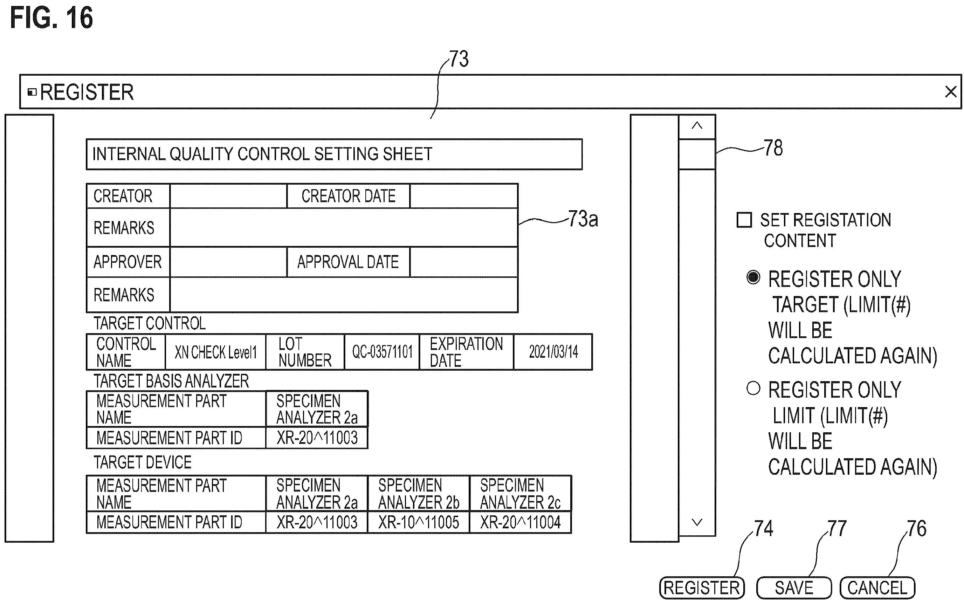

In the tool bar region 60, a button 61 for switching the mode of the chart graph region 90 to the chart graph displaying mode, a button 71 for switching the mode of the chart graph region 90 to the percentage graph displaying mode, a button 72 for switching the reference value setting assistance screen to an internal quality control setting sheet screen shown in FIG. 16, and the like are displayed. In the material region 80, the concentration level, the lot number, and the measurement result registration date of the quality control sample, the names of the specimen analyzers 2a, 2b, and 2c for which control reference values are generated, the number of times of measurement of the quality control sample, and the number of measurement days of the quality control sample, are displayed.

In the chart graph region 90, a target value displaying region 62 for displaying a generated target value for each of the specimen analyzers 2a, 2b, and 2c, a limit value displaying region 63 for displaying generated limit values for each of the specimen analyzers 2a, 2b, and 2c, and a chart graph region 64 for displaying a chart graph, are displayed.

The vertical axis of the chart graph region 64 indicates the magnitude of the RBC. A horizontal center line 69 of the chart graph region 64 indicates a target value provided by the manufacturer of the quality control sample. A region 65 is a region indicating control reference values for the specimen analyzer 2a, a dot 65a indicates a target value calculated in step S213, each of two dotted lines 65b indicates a limit value calculated in step S214 (in this example, 3 SD based on the target value is the limit value), and a vertical line 65c indicates the range of 2SD based on the target value. Regions 66 and 67 are regions indicating control reference values for the specimen analyzers 2b and 2c, respectively, and indicate target values, limit values, and the like in the same manner as the region 65. A region 68 is a region indicating a target value generated on the basis of the measurement results obtained from all of the specimen analyzers 2a, 2b, and 2c and indicates the average value of the measurement results and the range of 2SD based on the average value. The indications in the regions 65 to 68 make it possible for the user to take a comprehensive view to ascertain the control reference values for the specimen analyzers 2a, 2b, and 2c.

The horizontal axis on a right half of the chart graph region 64 indicates the number of times of measurement of the quality control sample and indicates the first time of measurement, the second time of measurement, the third time of measurement, and the subsequent times of measurement in order from the left. Three line graphs 70 are shown respectively for the specimen analyzers 2a, 2b, and 2c. The plot height position of each of the line graphs indicates the magnitude of the RBC. The line graphs 70 are displayed in the respective colors specified in the column "DISPLAY COLOR" in FIG. 7. The colors enable easy distinguishment as to which line graphs correspond to which specimen analyzers. The indications of the line graphs 70 make it possible for the user to ascertain changes in the measurement results from the specimen analyzers 2a, 2b, and 2c through comparison thereamong, whereby the user can ascertain variation and fluctuation tendencies of the measurement results.

If the button 71 is selected in the tool bar region 60 in FIG. 14, the mode of the chart graph region 90 is switched from the chart graph displaying mode to the percentage graph displaying mode shown in FIG. 15.

FIG. 15 shows an example of the reference value setting assistance screen displayed as a result of switching the mode of the chart graph region 90 to the percentage graph displaying mode. In the chart graph region 90, a coefficient-of-variation graph 75 is displayed. The vertical axis of the coefficient-of-variation graph 75 indicates the magnitude of coefficient of variation. In the coefficient-of variation graph 75, line graphs 75a, 75b, and 75c are respectively displayed for the specimen analyzers 2a, 2b, and 2c. Each of the line graphs 75a, 75b, and 75c has thereon rectangles the insides of which are white. Each of the rectangles indicates a coefficient of variation of measurement results of a quality control sample of a previous lot. The leftmost rectangle is of the oldest lot. Each of the line graphs 75a, 75b, and 75c also has thereon a rectangle the inside of which is colored. The rectangle indicates a coefficient of variation of the measurement results of the quality control sample of the new lot. The line graphs 75a, 75b, and 75c make it possible for the user to ascertain, for each of the specimen analyzers 2a, 2b, and 2c, the magnitudes of the coefficients of variation of the measurement results and changes in the coefficient of variation through comparison thereamong.

The user determines, on the basis of the content displayed in the reference value setting assistance screen in FIG. 14, FIG. 15, whether or not the target value and the limit value having been generated are appropriate. If the user determines that the values are appropriate, the user selects the button 72 in the tool bar region 60.

In step S216 in FIG. 12, the controller 31 of the computer 30a causes the display of the displaying-and-inputting unit 50 to display the internal quality control setting sheet.

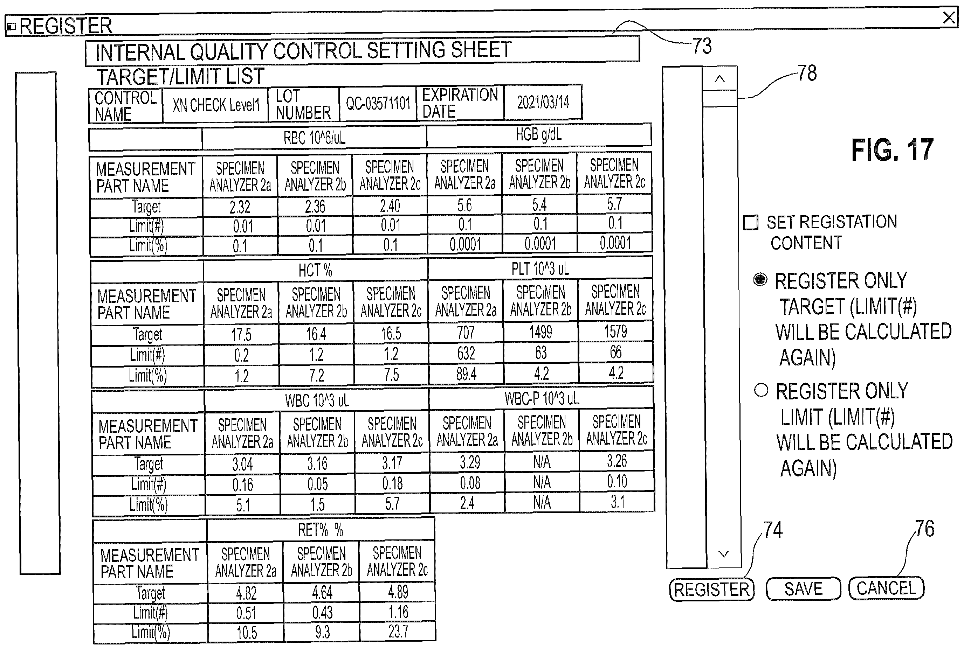

FIG. 16 and FIG. 17 show an example of the internal quality control setting sheet 73 displayed. The internal quality control setting sheet 73 is a sheet in which the generated control reference values are displayed in a format that supports an external certification such as an ISO certification. In addition, the internal quality control setting sheet 73 is stored in the storage unit 35 as an electronic file and can be printed by a printer.

FIG. 16 shows an upper half of the internal quality control setting sheet 73, and a lower half of the internal quality control setting sheet 73 shown in FIG. 17 is displayed by operating a scroll bar 78. In the upper half of the internal quality control setting sheet 73 shown in FIG. 16, a table 73a including items of a creator, a creation date, an approver, and an approval date is displayed in a format that enables the table 73a to be used also as a report. In the table 73a, characters can be inputted in a blank field of each of the items by using the keyboard of the displaying-and-inputting unit 50. Therefore, the user may input information such as a creator and an approver in the table 73a and print the internal quality control setting sheet 73, or may print the internal quality control setting sheet 73 with the table 73a being left blank and handwrite information such as a creator and an approver. As an example, in the table 73a, a creator may be inputted by using the keyboard of the displaying-and-inputting unit 50, and a creation date may be automatically inputted by the controller 31. In this case, the creator prints the internal quality control setting sheet 73, and an approver handwrites the name of the approver and the approval date.

Information about a quality control sample and specimen analyzers for which approval by the approver is to be performed is further displayed in the upper half of the internal quality control setting sheet 73. In the lower half of the internal quality control setting sheet 73 shown in FIG. 17, a target value and limit values having been generated are displayed for each of the specimen analyzers which are displayed for each of the measurement items.

In the displayed screen in FIG. 16 and FIG. 17, a checkbox indicating that registration content is to be set is displayed on the right side. If this checkbox is selected, either of the target values and the limit values can be registered alone. In a case where "REGISTER ONLY TARGET" is selected, the target values of the new lot each generated in step S213 are registered, and values previously applied to the specimen analyzer 2a are adopted as the limit values (%CV). In a case where "REGISTER ONLY LIMIT" is selected, the limit values (%CV) of the new lot each generated in step S214 are registered, and values previously applied to the specimen analyzer 2a are adopted as the target values. In either of the case of selecting "REGISTER ONLY TARGET" and the case of selecting "REGISTER ONLY LIMIT", each of the limit values (#) is calculated again on the basis of the corresponding target value of the new lot or the corresponding limit value (%CV) of the new lot.

If the creator determines that the control reference values are appropriate, the creator selects a save button through operation of the mouse of the displaying-and-inputting unit and stores the content of the internal quality control setting sheet in the storage unit 35. Then, the creator asks for approval by an approver. After obtaining approval by the approver, the creator selects a registration button 74 in the displayed screen in FIG. 16 and FIG. 17. Meanwhile, if the generated control reference values need to be checked again, the creator selects a cancel button 76 in the displayed screen in FIG. 16 or FIG. 17.

With reference back to FIG. 12, the controller 31 of the computer 30a determines, in step S217, whether the registration button 74 has been selected or the cancel button 76 has been selected. If the registration button 74 has been selected (in step S217, "REGISTER"), the controller 31 of the computer 30a returns the process to the main routine in FIG. 4B and applies, in step S25, the target value generated in step S213 and the limit values generated in step S214 to the specimen analyzers 2a, 2b, and 2c. Specifically, the controller 31 of the computer 30a overwrites the target value in the column "TARGET VALUE" of the QC file (FIG. 5) stored in the storage unit 35 of each of the computers 30a, 30b, and 30c and overwrites the limit value (#) and limit value (#)÷target value in the respective columns "LIMIT VALUE (#)" and "RANGE (%)" of the said QC file. Consequently, the target value generated in step S213 and the limit values generated in step S214 become control reference values to be used for internal quality control. Further, the target value generated in step S213 and the limit values generated in step S214 become values to be read out in the processing in step S32 in FIG. 4C.

As described above, the controller 31 of the computer 30a outputs, for the user, the control reference values in a format that supports an external certification such as an ISO certification. Thus, the user can apply the control reference values to the devices after thoroughly checking information necessary for the certification. Therefore, operation can be performed efficiently according to the facility that obtains an external certification.

The above method for applying a control reference value to the specimen analyzers 2a, 2b, and 2c, the above specimen analyzers 2a, 2b, and 2c, and the above reference value applying program eliminate the need for operations such as input of measurement results of a quality control sample to the computers 30a, 30b, and 30c by a person in charge in a test facility and input of an obtained control reference value to the specimen analyzers 2a, 2b, and 2c by the person. Consequently, the control reference value can be more efficiently determined and applied to the specimen analyzers in the test facility.

In addition, in the above embodiment, the computer 30a applies a control reference value to the specimen analyzers 2a, 2b, and 2c. Consequently, it takes the user less time to perform an operation of applying the control reference value to each of the specimen analyzers 2a, 2b, and 2c.

In addition, in the above embodiment, the computer 30a receives setting of a rule for each of the concentration levels of a quality control sample. Consequently, an optimum control reference value can be set for each of the concentration levels (attributes) of the quality control sample according to the facility.

In addition, in the above embodiment, setting of a rule is received through selection of a checkbox (choice). Consequently, the user can easily determine a control reference value by only selecting a pattern corresponding to the facility.

In addition, in the above embodiment, if a first rule is selected, the computer 30a generates a control reference value on the basis of a plurality of measurement results. Consequently, a control reference value obtained through appropriate reflection of a device state can be obtained.

In addition, in the above embodiment, a range of measurement results to be used for generating a control reference value can be specified. Consequently, even in, for example, a facility in which a measurement result of a quality control sample obtained within, for example, a specific period is desired to be excluded in calculation of a control reference value, an operation corresponding to this facility can easily be performed.

In addition, in the above embodiment, if "USE AVERAGE VALUE REGARDING EACH ANALYZER" or "USE STATISTICAL VALUE REGARDING EACH ANALYZER" is selected as a first rule, individual control reference values are generated for the respective specimen analyzers 2a, 2b, and 2c. Consequently, even in a facility in which operation is desired to be performed with different control reference values being set for the respective specimen analyzers 2a, 2b, and 2c, an operation corresponding to this facility can easily be performed.

In addition, in the above embodiment, if "USE AVERAGE VALUE REGARDING BASIS ANALYZER" or "USE STATISTICAL VALUE REGARDING BASIS ANALYZER" is selected as a first rule, a control reference value generated for the specimen analyzer 2a which is a basis analyzer can be applied as a control reference value common to the specimen analyzers 2a, 2b, and 2c. Consequently, even in a facility in which a control reference value obtained from a basis analyzer is desired to be applied to other analyzers in the facility, an operation corresponding to this facility can easily be performed.

In addition, in the above embodiment, if "USE AVERAGE VALUE REGARDING ALL ANALYZERS" or "USE STATISTICAL VALUE REGARDING ALL ANALYZERS" is selected as a first rule, a control reference value generated on the basis of measurement results obtained from all of the specimen analyzers 2a, 2b, and 2c can be applied as a control reference value common to the specimen analyzers 2a, 2b, and 2c. Consequently, even in a facility in which a control reference value obtained regarding all of the plurality of specimen analyzers 2a, 2b, and 2c in the facility is desired to be applied to each of the specimen analyzers 2a, 2b, and 2c in the facility, an operation corresponding to this facility can easily be performed.

In addition, in the above embodiment, if a first rule is selected, measurement results obtained from a quality control sample of a production lot (previous lot) preceding a new lot can be used for calculating a control reference value. Consequently, even in, for example, a facility in which a control reference value obtained through reflection of a long-term variation among the measurement results is desired to be used, an operation corresponding to this facility can easily be performed.

In addition, in the above embodiment, if a first rule is selected, the computer 30a causes displaying of the reference value setting assistance screen including: a generated control reference value; and a plurality of measurement results used for generating the control reference value. Consequently, the user can check the generated control reference value and the plurality of measurement results used for generating the control reference value and can contemplate appropriateness of the generated control reference value.

In addition, in the above embodiment, a second rule of generating a control reference value on the basis of a value specified by the user can be selected. Consequently, even in a facility in which a control reference value based on the value specified by the user is desired to be used, an operation corresponding to this facility can be performed efficiently.

In addition, in the above embodiment, a third rule of generating a control reference value on the basis of a value specified by a manufacturer of a quality control sample can be selected. Consequently, even in a facility in which a control reference value based on the value specified by the manufacturer is desired to be used, an operation corresponding to this facility can be performed efficiently.

In addition, in the above embodiment, the computer 30a receives setting of a rule for each of the measurement items. Consequently, an operation corresponding to a facility can easily be performed. In the facility, different control reference values are desired to be used according to the measurement items as in, for example, a facility in which a value specified by a manufacturer is desired to be used for a specific measurement item, and meanwhile, a control reference value generated on the basis of measurement results is desired to be used for another measurement item. For example, there is also a facility in which a value specified by a manufacturer is used for a measurement item such as hemoglobin (HGB) for which a measurement value of a quality control sample is obtained, the measurement value being known to hardly fluctuate. Adaptation to such a facility can easily be achieved as well.

In the above embodiment, the reference value applying program is loaded to the computer 30a from a portable storage medium. However, from an external device communicably connected to the computer 30a by an electric communication line (either wired or wireless one), the reference value applying program may be loaded via the electric communication line.

In the above embodiment, the computer 30a is disposed in the facility in which the user is present. However, a configuration may be employed in which: the computer 30a is set in a cloud; and the measurement device 3a disposed in the facility and the computer 30a are connected to each other over the Internet.

In the above embodiment, the measurement device 3a and the computer 30a are formed as separate devices. However, an integrated device may be formed by incorporating the function of the computer 30a into the measurement device 3a.

In the above embodiment, the blood cell counter for counting blood cells contained in a blood specimen has been described. However, the specimen to be analyzed by the blood cell counter is not limited to blood and may be body fluids other than blood, such as lymph and celomic fluid. In this case, a configuration may be employed in which setting of a rule of determining a reference value is received for each type (attribute) of body fluid as a quality control sample.

In the above embodiment, the blood cell counter has been described. However, the specimen analyzer is not limited to the blood cell counter and may be another specimen analyzer for which quality control is to be conducted by using a quality control sample, such as a biochemical analyzer, an immuno analyzer, a blood coagulation analyzer, a urine analyzer, or a genetic analyzer.

In the above embodiment, the specimen analysis system 1 includes the plurality of specimen analyzers 2a, 2b, and 2c. However, without limitation thereto, a configuration may be employed in which the specimen analysis system includes only the specimen analyzer 2a.

In the above embodiment, the computer 30a can receive setting of the first rules, the second rules, and the third rules. However, without limitation thereto, the computer 30a may be configured to receive only the first rules. In addition, the computer 30a may be configured to automatically store the first rules at the time of installation of the reference value applying program and not to receive rule setting.

## Claims

1. A method for applying a control reference value regarding a quality control sample to a specimen analyzer by a computer, the method comprising:
generating the control reference value on the basis of a measurement result of the quality control sample measured by the specimen analyzer; and
applying the control reference value to the specimen analyzer.

2. The method, for applying the control reference value to the specimen analyzer, of claim 1, the method further comprising
receiving setting of a rule of generating the control reference value on the basis of the measurement result, wherein
the generating of the control reference value comprises generating the control reference value based on the measurement result on the basis of the rule.

3. The method, for applying the control reference value to the specimen analyzer, of claim 2, wherein
the receiving of the setting of the rule comprises receiving setting of the rule for each of attributes of the quality control sample.

4. The method, for applying the control reference value to the specimen analyzer, of claim 3, wherein
the attributes of the quality control sample include an attribute regarding a concentration of a predetermined component contained in the quality control sample.

5. The method, for applying the control reference value to the specimen analyzer, of any one of claims 2 to 4, wherein
the setting of the rule comprises selecting a choice for setting the rule.

6. The method, for applying the control reference value to the specimen analyzer, of any one of claims 2 to 5, wherein
the rule includes a rule of generating the control reference value on the basis of a plurality of measurement results having been obtained by measuring the quality control sample a plurality of times,
obtaining of the measurement result comprises obtaining the plurality of measurement results, and
the generating of the control reference value comprises generating the control reference value based on at least one of the plurality of measurement results.

7. The method, for applying the control reference value to the specimen analyzer, of claim 6, wherein the rule includes a rule regarding specifying of a range of measurement results to be used for the generating of the control reference value among the plurality of measurement results having been obtained.

8. The method, for applying the control reference value to the specimen analyzer, of any one of claims 2 to 7, wherein
the applying of the control reference value comprises applying the control reference value to a plurality of specimen analyzers including the specimen analyzer, and
the rule includes a rule of
generating, on the basis of a measurement result obtained by measuring the quality control sample by the specimen analyzer, the control reference value that is to be applied to the specimen analyzer, and
generating, on the basis of a measurement result obtained by measuring the quality control sample by another specimen analyzer among the plurality of specimen analyzers, the control reference value that is to be applied to the other specimen analyzer.

9. The method, for applying the control reference value to the specimen analyzer, of any one of claims 2 to 8, wherein
the applying of the control reference value comprises applying the control reference value to a plurality of specimen analyzers including the specimen analyzer, and
the rule includes a rule of generating, on the basis of a measurement result obtained by measuring the quality control sample by any specimen analyzer among the plurality of specimen analyzers, the control reference value that is to be applied in common to the plurality of specimen analyzers.

10. The method, for applying the control reference value to the specimen analyzer, of any one of claims 2 to 9, wherein
the applying of the control reference value comprises applying the control reference value to a plurality of specimen analyzers including the specimen analyzer, and
the rule includes a rule of generating, on the basis of a plurality of measurement results obtained by measuring the quality control sample by the plurality of respective specimen analyzers, the control reference value that is to be applied in common to the plurality of specimen analyzers.

11. The method, for applying the control reference value to the specimen analyzer, of any one of claims 2 to 10, wherein
the rule includes a rule regarding whether or not a measurement result obtained from a quality control sample of a production lot preceding a production lot of the quality control sample is to be used for the generating of the control reference value.

12. The method, for applying the control reference value to the specimen analyzer, of any one of claims 2 to 11, wherein
the rule further includes a second rule of generating the control reference value on the basis of a value specified by a user,
when the rule of generating the control reference value on the basis of the measurement result of the quality control sample is set, the generating of the control reference value is executed, and
when the second rule is set, the method further comprises receiving input of the value specified by the user, and the applying of the control reference value comprises applying the value specified by the user to the specimen analyzer.

13. The method, for applying the control reference value to the specimen analyzer, of any one of claims 2 to 12, wherein
the rule further includes a third rule of generating the control reference value on the basis of a value specified by a manufacturer of the quality control sample,
when the rule of generating the control reference value on the basis of the measurement result of the quality control sample is set, the generating of the control reference value is executed, and
when the third rule is set, the method further comprises obtaining the value specified by the manufacturer, and the applying of the control reference value comprises applying the value specified by the manufacturer to the specimen analyzer.

14. The method, for applying the control reference value to the specimen analyzer, of any one of claims 2 to 13, wherein
the receiving of the setting of the rule comprises receiving setting of the rule for each of at least some measurement items among a plurality of measurement items.

15. The method, for applying the control reference value to the specimen analyzer, of any one of claims 2 to 14, wherein
the control reference value includes a desired value and a value indicating an allowable range.

16. The method, for applying the control reference value to the specimen analyzer, of claim 15, wherein
the receiving of the setting of the rule comprises receiving setting of the rule for each of the desired value and the allowable range.

17. The method, for applying the control reference value to the specimen analyzer, of any one of claims 1 to 16, wherein
the applying of the control reference value comprises applying the control reference value to a plurality of specimen analyzers including the specimen analyzer.

18. The method, for applying the control reference value to the specimen analyzer, of any one of claims 1 to 17, the method further comprising
displaying, when the control reference value is generated on the basis of the measurement result, screen information including the generated control reference value and the measurement result.

19. The method, for applying the control reference value to the specimen analyzer, of any one of claims 1 to 18, the method further comprising
outputting the generated control reference value in a format that supports an external certification, and
the applying of the control reference value is executed when input for registration of the outputted control reference value is received.

20. The method, for applying the control reference value to the specimen analyzer, of claim 19, wherein
the format includes an item of a creation date, and
the creation date is automatically generated.

21. The method, for applying the control reference value to the specimen analyzer, of any one of claims 1 to 20, wherein
the applying of the control reference value comprises storing, in the computer, history information including: a name of a user who has given an instruction to execute the applying; and a date and a time of the execution.

22. The method, for applying the control reference value to the specimen analyzer, of any one of claims 1 to 21, the method further comprising
obtaining the measurement result of the quality control sample, wherein
the generating of the control reference value comprises generating the control reference value on the basis of the obtained measurement result of the quality control sample.

23. A specimen analyzer to which a control reference value regarding a quality control sample is to be applied, the specimen analyzer comprising:
a measurement device; and
a computer connected to or incorporated in the measurement device, wherein
the computer
generates the control reference value on the basis of a measurement result of the quality control sample measured by the measurement device, and
applies the control reference value to the specimen analyzer.

24. A computer program configured to cause a computer, configured to apply a control reference value regarding a quality control sample to a specimen analyzer, to execute:
generating the control reference value on the basis of a measurement result of the quality control sample measured by the specimen analyzer; and
applying the control reference value to the specimen analyzer.
